# EUROPEAN PATENT SPECIFICATION

(11) **EP 3 326 456 B1**
(45) Date of publication and mention of the grant of the patent: **11.12.2019**
(21) Application number: 16870325.4
(22) Date of filing: 19.10.2016
(51) Int. Cl.: B01J 20/28, B01J 20/32

(54) **METHOD FOR PRODUCING WATER ABSORPTION TREATMENT MATERIAL**
VERFAHREN ZUR HERSTELLUNG VON WASSERAUFNAHMEBEHANDLUNGSMATERIAL
PROCÉDÉ DE PRODUCTION DE MATÉRIAU DE TRAITEMENT D'ABSORPTION D'EAU

(30) Priority: 30.11.2015 JP 2015232616
(43) Date of publication of application: 30.05.2018
(73) Proprietor: Daiki Co., Ltd., Minato-ku Tokyo 107-0052 (JP)
(72) Inventor: YOSHINAGA, Junji, Tokyo 107-0052 (JP)
(74) Representative: Müller-Boré & Partner Patentanwälte PartG mbB
(86) International application number: PCT/JP2016/080995
(87) International publication number: WO 2017/094385

(56) References cited:
- EP-A2- 1 161 860
- JP-A- 2015 042 147
- JP-A- 2015 097 996
- JP-B2- 3 011 899
- JP-B2- 4 274 884
- US-A1- 2005 000 463

## Description

### Technical Field

The present invention relates to a method for manufacturing a water absorption treatment material that absorbs a liquid.

### Background Art

Patent Document JP 2015-97996 A describes an example of a conventional water absorption treatment material. The water absorption treatment material is provided with a core portion formed into a granular shape and a coating portion that covers the core portion. The coating portion has a function of adhering pieces of the water absorption treatment material together, which have absorbed liquid during use. Accordingly, a clump composed of multiple pieces of the water absorption treatment material is formed after use.

JP 2015 042147 A discloses a water absorbing treatment material for absorbing liquid including a granular core part, and a covering layer. The granular core part includes fluff pulp and a pigmented plastic. The fluff pulp and the pigmented plastic are obtained by classifying a sanitary article. The covering layer covers the granular core part.

EP 1 161 860 A2 discloses an animal excretions-treating material including particles, and each particle is composed of a core layer of fibers and a skin layer to cover the core layer. The skin layer contains alpha -starch and fibers.

### Summary of Invention

### Technical Problem

Thus, the coating portion contributes to forming a clump of the water absorption treatment material after use. However, on the other hand, since the coating portion is provided so as to cover the core portion, it inhibits the liquid from quickly reaching the core portion. This has caused the water-absorptivity to decrease in the conventional water absorption treatment material.

The present invention is defined by independent claim 1. Preferred embodiments are defined in the dependent claims. In particular, there is provided a method for manufacturing a water absorption treatment material, according to which it is possible to suppress a decrease in water-absorptivity caused by the existence of the coating portion.

### Solution to the Problem

A method for manufacturing a water absorption treatment material according to the present invention includes: a core portion forming step of forming a core portion in a granular shape; a coating portion forming step of forming a coating portion so as to cover the core portion; and a coating portion removing step of removing a part of the coating portion.

With this manufacturing method, after the coating portion is formed on the core portion, a part of the coating portion is removed. For this reason, with the manufactured water absorption treatment material, the liquid can quickly reach the core portion through the portion where the coating portion has been removed.

### Advantageous Effects of Invention

According to the present invention, a method for manufacturing a water absorption treatment material is realized, according to which it is possible to suppress a decrease in water-absorptivity caused by the existence of the coating portion.

### Brief Description of Drawings

FIG. 1 is a perspective view showing an embodiment of a water absorption treatment material according to the present invention.
FIG. 2 is a cross-sectional view taken along line II-II in FIG. 1.
FIG. 3 is a perspective view showing a core portion of the water absorption treatment material shown in FIG. 1.
FIG. 4 is a cross-sectional view illustrating a coating portion forming step.
FIG. 5 is a cross-sectional view illustrating a coating portion removing step.

### Description of Embodiments

Hereinafter, an embodiment of the present invention will be described in detail with reference to the drawings. Note that in the description of the drawings, like elements are denoted by like reference numerals and redundant description thereof is not included.

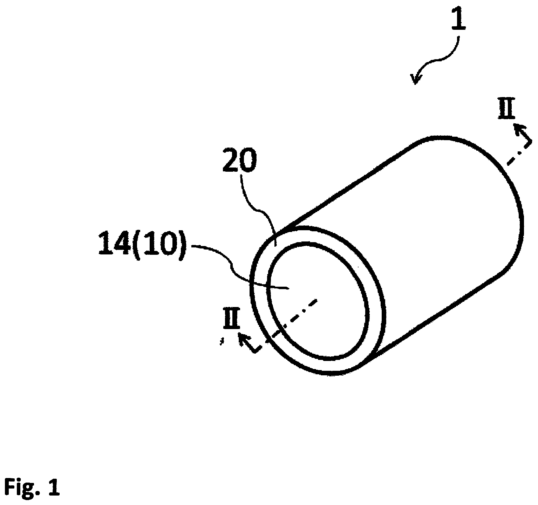

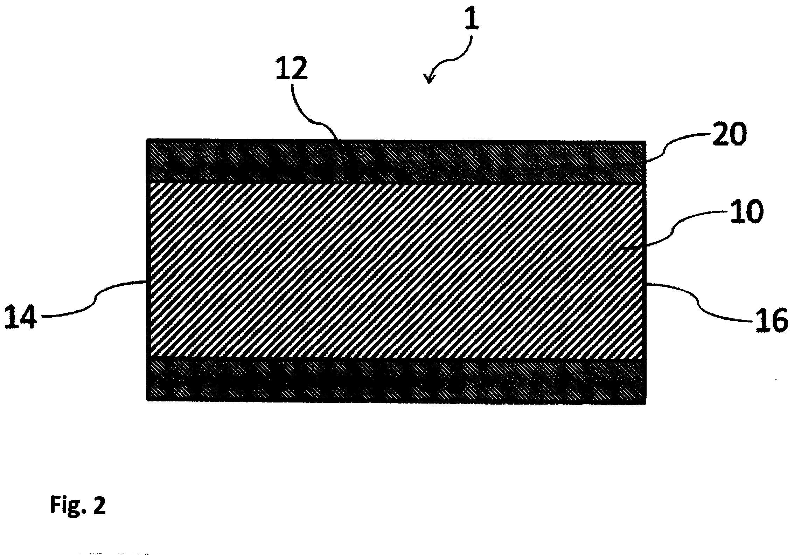

FIG. 1 is a perspective view showing an embodiment of a water absorption treatment material according to the present invention. Also, FIG. 2 is a cross-sectional view taken along line II-II in FIG. 1. A water absorption treatment material 1 is a water absorption treatment material that absorbs a liquid, and includes a core portion 10 and a coating portion 20. The water absorption treatment material 1 is an excrement treatment material that absorbs human or animal excrement, for example.

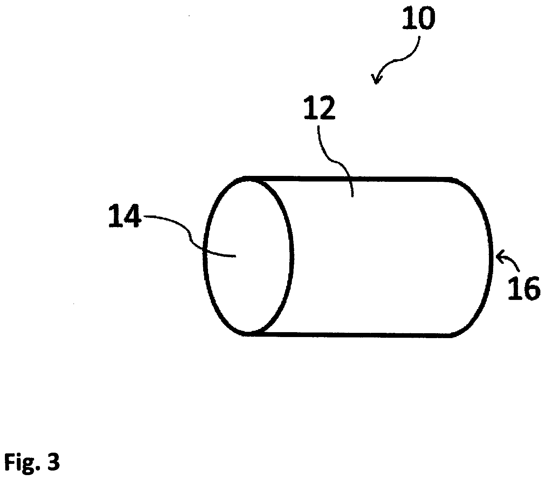

FIG. 3 is a perspective view showing the core portion 10. The core portion 10 is in a granular shape. In the present embodiment, the core portion 10 is approximately circular column-shaped and has a side surface 12, a bottom surface 14 (first bottom surface), and a bottom surface 16 (second bottom surface). The core portion 10 is water-absorbent and has a function of absorbing and retaining liquids such as urine. The core portion 10 contains a water-absorptive material (first water-absorptive material). For example, paper, tea grounds, plastic, or bean curd lees can be used as the first water-absorptive material. The first water-absorptive material is preferably the main material of the core portion 10. Here, the main material of the core portion 10 refers to a material that has the largest weight ratio in the core portion 10 among materials constituting the core portion 10.

Paper refers to a material mainly composed of pulp. Examples of paper include classified vinyl chloride wallpaper (paper obtained by classifying vinyl chloride wallpaper), fluff pulp, papermaking sludge, and pulp sludge, in addition to normal paper. It is also possible to use classified paper diapers (plastic obtained by classifying paper diapers) as the plastic, for example. The bean curd lees are preferably dried bean curd lees.

Returning to FIGS. 1 and 2, the coating portion 20 is provided so as to cover the core portion 10. The coating portion 20 has a function of adhering pieces of the water absorption treatment material 1 together, which have absorbed liquid during use. Accordingly, a clump composed of multiple pieces of the water absorption treatment material 1 is formed after use.

The coating portion 20 covers only a portion of the core portion 10. It is preferable that a region of 80% or more of the side surface 12 of the core portion 10 is covered by the coating portion 20. That is, it is preferable that the area of the portion of the side surface 12 that is covered by the coating portion 20 is 80% or more of the area of the entire side surface 12. FIGS. 1 and 2 show cases in which the entirety of the side surface 12 is covered by the coating portion 20.

On the other hand, it is preferable that a region of 80% or more of the bottom surface 14 of the core portion 10 is exposed without being covered by the coating portion 20. That is, it is preferable that the area of the portion of the bottom surface 14 that is exposed without being covered by the coating portion 20 is 80% or more of the area of the entire bottom surface 14. FIGS. 1 and 2 show cases in which the entirety of the bottom surface 14 is exposed without being covered by the coating portion 20.

Similarly, it is preferable that a region of 80% or more of the bottom surface 16 of the core portion 10 is exposed without being covered by the coating portion 20. That is, it is preferable that the area of the portion of the bottom surface 16 that is exposed without being covered by the coating portion 20 is 80% or more of the area of the entire bottom surface 16. FIG. 2 shows a case in which the entirety of the bottom surface 16 is exposed without being covered by the coating portion 20.

Note that although unevenness and cracks are sometimes present in the side surface 12, such unevenness and the like is ignored when considering the area ratio of the portion covered by the coating portion 20. Specifically, it is sufficient to consider the area ratio on a circular tube surface when the side surface 12 is projected onto the circular tube surface having the same central axis as the core portion 10. The same applies to the bottom surfaces 14 and 16. That is, it is sufficient to consider the area ratio on a plane when the bottom surfaces 14 and 16 are projected onto the planes orthogonal to the central axis.

The coating portion 20 contains a water-absorptive material (second water-absorptive material) and an adhesive material. For example, paper, tea grounds, plastic, or bean curd lees can be used as the second water-absorptive material as well. The second water-absorptive material may be the same as or different from the first water-absorptive material. The second water-absorptive material is preferably the main material of the coating portion 20. The definition of the main material of the coating portion 20 is the same as the above-described definition of the main material of the core portion 10. For example, water-absorptive polymer, starch, CMC (carboxymethyl cellulose), PVA (polyvinyl alcohol), or dextrin can be used as the adhesive material.

Next, an example of a method for manufacturing the water absorption treatment material 1 will be described as an embodiment of a method for manufacturing a water absorption treatment material according to the present invention. The manufacturing method includes a core portion forming step, a coating portion forming step, and a core portion removing step.

The core portion forming step is a step of forming the core portion 10 in a granular shape. In this step, the core portion 10 is formed through extrusion granulation. Specifically, the material (core portion material) constituting the core portion 10 is pulverized to a predetermined size by a pulverizer, and thereafter is introduced into a mixer at a predetermined ratio and is mixed together. Then, water is added according to need, and thereafter the core portion material is subjected to extrusion granulation by a granulator. Accordingly, the core portion 10 in an approximately circular column shape is obtained.

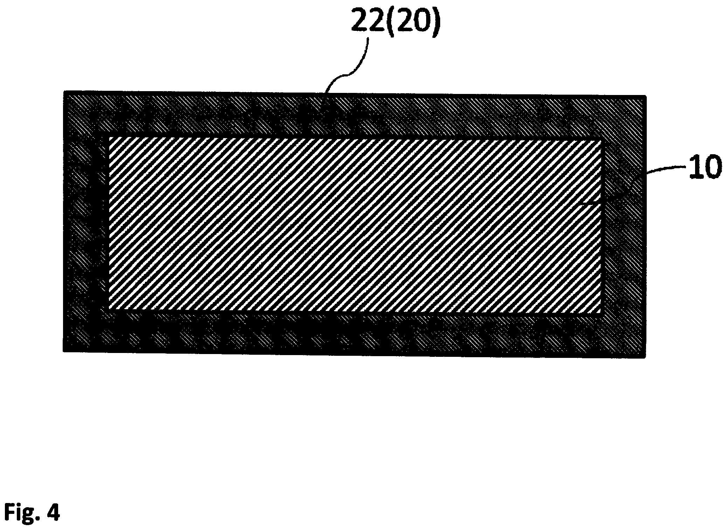

The coating portion forming step is a step of forming the coating portion 20 so as to cover the core portion 10. In this step, as shown in FIG. 4, a coating material 22, which is a material that constitutes the coating portion 20, is attached to the core portion 10. At this time, the coating material 22 may be attached to the entirety or a portion of the periphery of the core portion 10. FIG. 4 shows a case in which the coating material 22 is attached to the entire periphery of the core portion 10. For example, a coating apparatus can be used to attach the coating material 22. Thus, the coating portion 20 is formed on the side surface 12, the bottom surface 14 and the bottom surface 16.

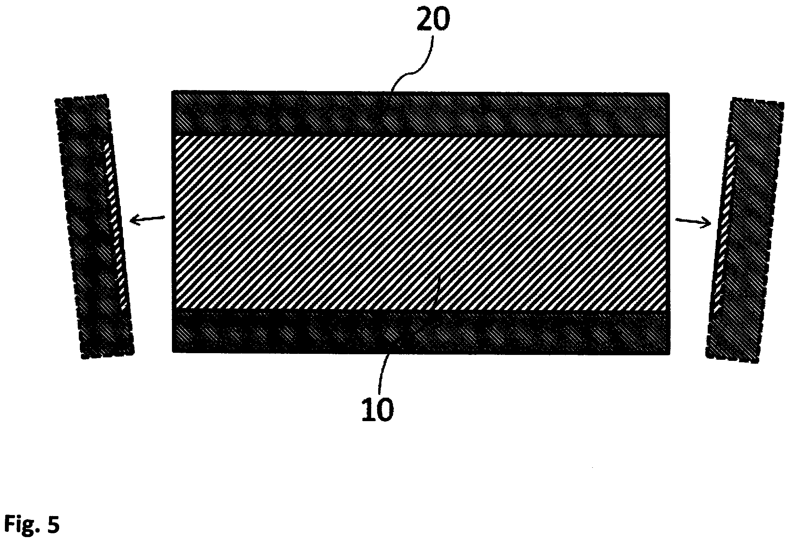

The coating portion removing step is a step of removing a part of the coating portion 20 formed in the coating portion forming step. In this step, as shown in FIG. 5, an end portion of the core portion 10 is cut off. In the present embodiment, both of the end portion on the bottom surface 14 side and the end portion on the bottom surface 16 side are cut off. Thus, the coating portion 20 formed on the bottom surface 14 is removed, and the coating portion 20 formed on the bottom surface 16 is removed. At this time, it is sufficient to remove at least a part of the coating portion 20 formed on each of the bottom surfaces 14 and 16. The core portion 10 is exposed at the portions where the coating portion 20 is removed in this manner.

In the coating portion removing step, it is preferable that the coating portion 20 is removed such that a region of 10% or more of the periphery of the core portion 10 is exposed, and it is more preferable that the coating portion 20 is removed such that a region of 30% or more of the periphery of the core portion 10 is exposed.

Also, it is preferable that the coating portion 20 is removed such that a region of 80% or more of each of the bottom surfaces 14 and 16 is exposed without being covered by the coating portion 20, while the coating portion 20 is left on a region of 80% or more of the side surface 12. FIG. 5 shows a case in which the coating portion 20 is removed such that the entirety of each of the bottom surfaces 14 and 16 is exposed without being covered by the coating portion 20, while the coating portion 20 is left on the entirety of the side surface 12.

Thereafter, only granular bodies that satisfy a predetermined standard are extracted by sieving the granular bodies obtained in this manner (size separation step). Then, the extracted granular bodies are dried using a dryer (drying step). The moisture content of the core portions 10 is adjusted as appropriate through the drying. According to the above description, the water absorption treatment material 1 is obtained.

Effects of the present embodiment will be described hereinafter. In the present embodiment, after the coating portion 20 is formed on the core portion 10, a part of the coating portion 20 is removed. For this reason, in the water absorption treatment material 1, the liquid can quickly reach the core portion 10 through the portion where the coating portion 20 has been removed. Accordingly, the water absorption treatment material 1 and the method for manufacturing the same, according to which it is possible to suppress a decrease in water-absorptivity caused by the existence of the coating portion 20, are realized.

By removing a part of the coating portion 20 after forming thereof in this manner, the coating portion 20 that partially covers the core portion 10 can be easily obtained. Also, the coating portion 20 that partially covers the core portion 10 can ultimately be obtained even if the coating material 22 is attached to the entire periphery of the core portion 10 in the coating portion forming step. In actuality, in the present embodiment, the coating material 22 is attached to the entire periphery of the core portion 10. For this reason, there is an advantage in that the coating material 22 can be attached to the core portion 10 without using a special apparatus or method.

In the coating portion removing step, the coating portion 20 is removed such that the core portion 10 is exposed at the portions where the coating portion 20 has been removed (the bottom surfaces 14 and 16). By exposing the core portion 10 in this manner, the liquid can more quickly reach the core portion 10 in the water absorption treatment material 1. From the viewpoint of sufficiently extracting this effect, it is preferable that the coating portion 20 is removed such that a region of 10% or more of the periphery of the core portion 10 is exposed, and it is more preferable that the coating portion 20 is removed such that a region of 30% or more of the periphery of the core portion 10 is exposed.

In the coating portion removing step, the coating portion 20 formed on each of the bottom surfaces 14 and 16 of the core portion 10 is removed. Thus, the configuration in which a part of the coating portion 20 has been removed can be easily realized. In actuality, in the present embodiment, the coating portion 20 is removed by the simple means of cutting off the end portions of the core portion 10.

Exposing the majority of each of the bottom surfaces 14 and 16 is advantageous for increasing the area of contact with the liquid of the core portion 10, whereby the water-absorptivity (in particular, the water absorption speed) of the water absorption treatment material 1 is improved. From this viewpoint, it is preferable that the coating portion 20 is removed such that a region of 80% or more of each of the bottom surfaces 14 and 16 is exposed without being covered by the coating portion 20, and it is more preferable that the coating portion 20 is removed such that the entirety of each of the bottom surfaces 14 and 16 is exposed without being covered by the coating portion 20.

Also, leaving the coating portion 20 on the majority of the side surface 12 is advantageous for sufficiently adhering the pieces of the water absorption treatment material 1 together, which have absorbed the liquid during use. Moreover, in this case, the majority of the side surface 12, which tends to be more noticeable compared to the bottom surfaces 14 and 16, is covered by the coating portion 20, and therefore there is an advantage in that the aesthetics of the water absorption treatment material 1 are not impaired. From these viewpoints, in the coating portion removing step, it is preferable that the coating portion 20 is left on a region of 80% or more of the side surface 12, and it is more preferable that the coating portion 20 is left on the entirety of the side surface 12.

In the core portion forming step, the core portion 10 is formed through extrusion granulation. Accordingly, the core portion 10 in a granular shape (approximately circular column shape) can be easily obtained.

Incidentally, the used water absorption treatment material 1 needs to be disposed of, and convenience for the user is increased if disposal can be performed by flushing the water absorption treatment material 1 in a flushing toilet. In order to perform disposal by thus flushing in a flushing toilet, it is required that the water absorption treatment material 1 has sufficient water-solubility (a property in which bonded fibers and particles rapidly separate and dissolve in water due to coming into contact with water).

In this respect, the conventional water absorption treatment material has had a problem in that the core portion is covered by the coating portion, which incurs a decrease in water-absorptivity as well as a decrease in water-solubility. This is because the coating portion blocks the water of the flushing toilet from reaching the core portion. In the present embodiment, a part of the coating portion 20 is removed as described above, and therefore this problem can be solved.

If the adhesive material contained in the coating portion is a water-absorptive polymer, the problem of the decrease in water-solubility of the conventional water absorption treatment material is prominent. This is because water-absorptive polymers have a property of expanding during liquid absorption, and when the water-absorptive polymer swells in the coating portion, the water of the flushing toilet is further prevented from reaching the core portion. For this reason, in such a case, a water absorption treatment material 1 that can prevent a decrease in water-solubility is particularly useful.

The present invention is not limited to the above-described embodiment, and various modifications are possible. The above-described embodiment illustrated a case in which both end portions of the core portion 10 are cut off. However, only one end portion (the end portion on the bottom surface 14 side or the end portion on the bottom surface 16 side) of the core portion 10 may be cut off.

The above-described embodiment illustrated a case in which the coating portion 20 on the bottom surfaces 14 and 16 of the core portion 10 is removed. However, in the coating portion removing step, the coating portion 20 may be removed on an arbitrary position of the core portion 10 as long as a part of the coating portion 20 is removed. For example, at least a part of the coating portion 20 on the side surface 12 may be removed.

The above-described embodiment illustrated a case in which the core portion 10 is exposed at the portion where the coating portion 20 has been removed. However, exposing the core portion 10 at that portion is not essential. Even in a case in which the coating portion 20 is removed to such an extent that the core portion 10 is not exposed, a portion of the coating portion 20 becomes thinner, thus the liquid can be quickly introduced to the core portion 10 through that portion.

The above-described embodiment illustrated a case in which not only a part of the coating portion 20 but also a part of the core portion 10 is cut off. However, only the coating portion 20 may be cut off without cutting off the core portion 10.

The above-described embodiment illustrated the method (cutting-off method) of removing the coating portion 20 by cutting off a part of the core portion 10 on which the coating portion 20 has been formed. However, a method other than the cutting-off method can be used for removing the coating portion 20. Examples of the method other than the cutting-off method include the method (collision method) of removing the coating portion 20 by having a plurality of the core portions 10 on which the coating portion 20 has been formed collide against one another, or the method (rubbing method) of removing the coating portion 20 by having a plurality of the core portions 10 on which the coating portion 20 has been formed rub against one another.

In the collision method, a part of the coating portion 20 is removed by the impact force applied when the plurality of the core portions 10 collide against one another. In the rubbing method, a part of the coating portion 20 is removed by the friction force applied when the plurality of the core portions 10 rub against one another. In the case in which these methods are used, a plurality of the core portions 10 are formed in the core portion forming step, and the coating portion 20 is formed so as to cover each of the core portions 10 in the coating portion forming step. In the case in which the collision method or the rubbing method is used, there is an advantage in that the coating portions 20 are simultaneously removed from the plurality of the core portions 10. Note that in the coating portion removing step, any two methods of the cutting-off method, the collision method and the rubbing method may be executed, or all of the three methods may be executed.

The above-described embodiment illustrated a case in which the core portion 10 is in a circular column shape. However, the shape of the core portion 10 is not limited to a circular column, and may be a sphere, an ellipsoid or the like.

### List of Reference Numerals

- 1: Water absorption treatment material
- 10: Core portion
- 12: Side surface
- 14: Bottom surface (first bottom surface)
- 16: Bottom surface (second bottom surface)
- 20: Coating portion
- 22: Coating material

## Claims

1. A method for manufacturing a water absorption treatment material (1), comprising:
a core portion forming step of forming a core portion (10) in a granular shape;
a coating portion forming step of forming a coating portion (20) so as to cover the core portion (10); and
a coating portion removing step of removing a part of the coating portion (20),
and
wherein in the coating portion removing step, the coating portion (20) is removed such that the core portion (10) is exposed at a portion where the coating portion (20) is removed.

2. The method for manufacturing a water absorption treatment material (1) according to claim 1,
wherein in the coating portion forming step, the coating portion (20) is formed by attaching a coating material (22) to the core portion (10).

3. The method for manufacturing a water absorption treatment material (1) according to claim 2,
wherein in the coating portion forming step, the coating material (22) is attached to the entire periphery of the core portion (10).

4. The method for manufacturing a water absorption treatment material (1) according to one of claims 1 to 3,
wherein in the core portion forming step, the core portion (10) in an approximately circular column shape having a side surface (12) and first and second bottom surfaces is formed.

5. The method for manufacturing a water absorption treatment material (1) according to claim 4,
wherein in the core portion forming step, the core portion (10) is formed through extrusion granulation.

6. The method for manufacturing a water absorption treatment material (1) according to claim 4 or 5,
wherein in the coating portion forming step, the coating portion (20) is formed on the side surface (12), and the first and second bottom surfaces of the core portion (10).

7. The method for manufacturing a water absorption treatment material (1) according to claim 6,
wherein in the coating portion removing step, at least a part of the coating portion (20) formed on the first bottom surface (14) of the core portion (10) is removed.

8. The method for manufacturing a water absorption treatment material (1) according to claim 7,
wherein in the coating portion removing step, the coating portion (20) is removed such that a region of 80% or more of the first bottom surface (14) of the core portion (10) is exposed without being covered by the coating portion (20), while the coating portion (20) is left on a region of 80% or more of the side surface (12) of the core portion (10).

9. The method for manufacturing a water absorption treatment material (1) according to claim 8,
wherein in the coating portion removing step, the coating portion (20) is removed such that the entirety of the first bottom surface (14) of the core portion (10) is exposed without being covered by the coating portion (20), while the coating portion (20) is left on the entirety of the side surface (12) of the core portion (10).

10. The method for manufacturing a water absorption treatment material (1) according to one of claims 7 to 9,
wherein in the coating portion removing step, the coating portion (20) formed on the first bottom surface (14) is removed by cutting off an end portion on the first bottom surface (14) side of the core portion (10).

11. The method for manufacturing a water absorption treatment material (1) according to one of claims 6 to 10,
wherein in the coating portion removing step, at least a part of the coating portion (20) formed on the second bottom surface (16) of the core portion (10) is removed.

12. The method for manufacturing a water absorption treatment material (1) according to one of claims 1 to 11,
wherein in the core portion forming step, a plurality of the core portions (10) are formed, and
in the coating portion forming step, the coating portion (20) is formed so as to cover each of the core portions (10).

13. The method for manufacturing a water absorption treatment material (1) according to claim 12,
wherein in the coating portion removing step, the coating portion (20) is removed by having the plurality of the core portions (10) on which the coating portion (20) is formed collide against one another.

14. The method for manufacturing a water absorption treatment material (1) according to claim 12 or 13,
wherein in the coating portion removing step, the coating portion (20) is removed by having the plurality of the core portions (10) on which the coating portion (20) is formed rub against one another.

## Patentansprüche

1. Verfahren zum Herstellen eines Wasserabsorptionsbehandlungsmaterials (1), umfassend:
einen Kernabschnitt-Ausbildungsschritt zum Ausbilden eines Kernabschnitts (10) in einer körnigen Form;
einen Beschichtungsabschnitt-Ausbildungsschritt zum Ausbilden eines Beschichtungsabschnitts (20), um den Kernabschnitt (10) zu bedecken; und
einen Beschichtungsabschnitt-Entfernungsschritt zum Entfernen eines Teils des Beschichtungsabschnitts (20),
und
wobei in dem Beschichtungsabschnitt-Entfernungsschritt der Beschichtungsabschnitt (20) derart entfernt wird, dass der Kernabschnitt (10) an einem Abschnitt freigelegt wird, an dem der Beschichtungsabschnitt (20) entfernt wird bzw. ist.

2. Verfahren zum Herstellen eines Wasserabsorptionsbehandlungsmaterials (1) nach Anspruch 1,
wobei in dem Beschichtungsabschnitt-Ausbildungsschritt der Beschichtungsabschnitt (20) durch Anbringen eines Beschichtungsmaterials (22) an dem Kernabschnitt (10) ausgebildet wird.

3. Verfahren zum Herstellen eines Wasserabsorptionsbehandlungsmaterials (1) nach Anspruch 2,
wobei in dem Beschichtungsabschnitt-Ausbildungsschritt das Beschichtungsmaterial (22) an dem gesamten Umfang des Kernabschnitts (10) angebracht wird.

4. Verfahren zum Herstellen eines Wasserabsorptionsbehandlungsmaterials (1) nach einem der Ansprüche 1 bis 3,
wobei in dem Kernabschnitt-Ausbildungsschritt der Kernabschnitt (10) in einer ungefähr kreisförmigen Säulenform mit einer Seitenfläche (12) und einer ersten und einer zweiten Bodenfläche ausgebildet wird.

5. Verfahren zum Herstellen eines Wasserabsorptionsbehandlungsmaterials (1) nach Anspruch 4,
wobei in dem Kernabschnitt-Ausbildungsschritt der Kernabschnitt (10) durch Extrusionsgranulieren ausgebildet wird.

6. Verfahren zum Herstellen eines Wasserabsorptionsbehandlungsmaterials (1) nach Anspruch 4 oder 5,
wobei in dem Beschichtungsabschnitt-Ausbildungsschritt der Beschichtungsabschnitt (20) auf der Seitenfläche (12) und der ersten und der zweiten Bodenfläche des Kernabschnitts (10) ausgebildet wird.

7. Verfahren zum Herstellen eines Wasserabsorptionsbehandlungsmaterials (1) nach Anspruch 6,
wobei in dem Beschichtungsabschnitt-Entfernungsschritt zumindest ein Teil des Beschichtungsabschnitts (20), der auf der ersten Bodenfläche (14) des Kernabschnitts (10) ausgebildet ist, entfernt wird.

8. Verfahren zum Herstellen eines Wasserabsorptionsbehandlungsmaterials (1) nach Anspruch 7,
wobei in dem Beschichtungsabschnitt-Entfernungsschritt der Beschichtungsabschnitt (20) derart entfernt wird, dass ein Bereich von 80% oder mehr der ersten Bodenfläche (14) des Kernabschnitts (10) freigelegt wird, ohne von dem Beschichtungsabschnitt (20) bedeckt zu werden, während der Beschichtungsabschnitt (20) in einem Bereich von 80% oder mehr der Seitenfläche (12) des Kernabschnitts (10) verbleibt.

9. Verfahren zum Herstellen eines Wasserabsorptionsbehandlungsmaterials (1) nach Anspruch 8,
wobei in dem Beschichtungsabschnitt-Entfernungsschritt der Beschichtungsabschnitt (20) derart entfernt wird, dass die Gesamtheit der ersten Bodenfläche (14) des Kernabschnitts (10) freigelegt wird, ohne von dem Beschichtungsabschnitt (20) bedeckt zu werden, während der Beschichtungsabschnitt (20) auf der Gesamtheit der Seitenfläche (12) des Kernabschnitts (10) verbleibt.

10. Verfahren zum Herstellen eines Wasserabsorptionsbehandlungsmaterials (1) nach einem der Ansprüche 7 bis 9,
wobei in dem Beschichtungsabschnitt-Entfernungsschritt der auf der ersten Bodenfläche (14) ausgebildete Beschichtungsabschnitt (20) durch Abschneiden eines Endabschnitts auf der Seite der ersten Bodenfläche (14) des Kernabschnitts (10) entfernt wird.

11. Verfahren zum Herstellen eines Wasserabsorptionsbehandlungsmaterials (1) nach einem der Ansprüche 6 bis 10,
wobei in dem Beschichtungsabschnitt-Entfernungsschritt zumindest ein Teil des Beschichtungsabschnitts (20), der auf der zweiten Bodenfläche (16) des Kernabschnitts (10) ausgebildet ist, entfernt wird.

12. Verfahren zum Herstellen eines Wasserabsorptionsbehandlungsmaterials (1) nach einem der Ansprüche 1 bis 11,
wobei in dem Kernabschnitt-Ausbildungsschritt eine Mehrzahl der Kernabschnitte (10) ausgebildet wird, und
in dem Beschichtungsabschnitt-Ausbildungsschritt der Beschichtungsabschnitt (20) so ausgebildet wird, dass er jeden der Kernabschnitte (10) bedeckt.

13. Verfahren zum Herstellen eines Wasserabsorptionsbehandlungsmaterials (1) nach Anspruch 12,
wobei in dem Beschichtungsabschnitt-Entfernungsschritt der Beschichtungsabschnitt (20) entfernt wird, indem die Mehrzahl der Kernabschnitte (10), auf denen der Beschichtungsabschnitt (20) ausgebildet ist, gegeneinander kollidieren.

14. Verfahren zum Herstellen eines Wasserabsorptionsbehandlungsmaterials (1) nach Anspruch 12 oder 13,
wobei in dem Beschichtungsabschnitt-Entfernungsschritt der Beschichtungsabschnitt (20) entfernt wird, indem die Mehrzahl der Kernabschnitte (10), auf denen der Beschichtungsabschnitt (20) ausgebildet ist, aneinander reiben.

## Revendications

1. Procédé de fabrication d'un matériau de traitement d'absorption d'eau (1) comprenant :
une étape de formation de portion de cœur consistant à former une portion de cœur (10) en une forme granulaire ;
une étape de formation de portion d'enrobage consistant à former une portion d'enrobage (20) de manière à recouvrir la portion de cœur (10) ; et
une étape d'élimination de portion d'enrobage consistant à éliminer une partie de la portion d'enrobage (20),
et
dans lequel dans l'étape d'élimination de portion d'enrobage, la portion d'enrobage (20) est éliminée de sorte que la portion de cœur (10) est exposée au niveau d'une portion où la portion d'enrobage (20) est éliminée.

2. Procédé de fabrication d'un matériau de traitement d'absorption d'eau (1) selon la revendication 1,
dans lequel dans l'étape de formation de portion d'enrobage, la portion d'enrobage (20) est formée en reliant un matériau d'enrobage (22) à la portion de cœur (10).

3. Procédé de fabrication d'un matériau de traitement d'absorption d'eau (1) selon la revendication 2,
dans lequel dans l'étape de formation de portion d'enrobage, le matériau d'enrobage (22) est relié à la périphérie entière de la portion de cœur (10).

4. Procédé de fabrication d'un matériau de traitement d'absorption d'eau (1) selon une des revendications 1 à 3,
dans lequel dans l'étape de formation de portion de cœur, la portion de cœur (10) en une forme de colonne approximativement circulaire ayant une surface latérale (12) et des première et seconde surfaces inférieures est formée.

5. Procédé de fabrication d'un matériau de traitement d'absorption d'eau (1) selon la revendication 4,
dans lequel dans l'étape de formation de portion de cœur, la portion de cœur (10) est formée par granulation par extrusion.

6. Procédé de fabrication d'un matériau de traitement d'absorption d'eau (1) selon la revendication 4 ou 5,
dans lequel dans l'étape de formation de portion d'enrobage, la portion d'enrobage (20) est formée sur la surface latérale (12) et les première et seconde surfaces inférieures de la portion de cœur (10).

7. Procédé de fabrication d'un matériau de traitement d'absorption d'eau (1) selon la revendication 6,
dans lequel dans l'étape d'élimination de portion d'enrobage, au moins une partie de la portion d'enrobage (20) formée sur la première surface inférieure (14) de la portion de cœur (10) est éliminée.

8. Procédé de fabrication d'un matériau de traitement d'absorption d'eau (1) selon la revendication 7,
dans lequel dans l'étape d'élimination de portion d'enrobage, la portion d'enrobage (20) est éliminée de sorte qu'une région de 80 % ou plus de la première surface inférieure (14) de la portion de cœur (10) est exposée sans être recouverte de la portion d'enrobage (20) tandis que la portion d'enrobage (20) est laissée sur une région de 80 % ou plus de la surface latérale (12) de la portion de cœur (10).

9. Procédé de fabrication d'un matériau de traitement d'absorption d'eau (1) selon la revendication 8,
dans lequel dans l'étape d'élimination de portion d'enrobage, la portion d'enrobage (20) est éliminée de sorte que l'intégralité de la première surface inférieure (14) de la portion de cœur (10) est exposée sans être recouverte de la portion d'enrobage (20) tandis que la portion d'enrobage (20) est laissée sur l'intégralité de la surface latérale (12) de la portion de cœur (10).

10. Procédé de fabrication d'un matériau de traitement d'absorption d'eau (1) selon une des revendications 7 à 9,
dans lequel dans l'étape d'élimination de portion d'enrobage, la portion d'enrobage (20) formée sur la première surface inférieure (14) est éliminée en découpant une portion d'extrémité sur le côté de la première surface inférieure (14) de la portion de cœur (10).

11. Procédé de fabrication d'un matériau de traitement d'absorption d'eau (1) selon une des revendications 6 à 10,
dans lequel dans l'étape d'élimination de portion d'enrobage, au moins une partie de la portion d'enrobage (20) formée sur la seconde surface inférieure (16) de la portion de cœur (10) est éliminée.

12. Procédé de fabrication d'un matériau de traitement d'absorption d'eau (1) selon une des revendications 1 à 11,
dans lequel dans l'étape de formation de portion de cœur, une pluralité des portions de cœur (10) sont formées, et
dans l'étape de formation de portion d'enrobage, la portion d'enrobage (20) est formée de manière à recouvrir chacune des portions de cœur (10).

13. Procédé de fabrication d'un matériau de traitement d'absorption d'eau (1) selon la revendication 12,
dans lequel dans l'étape d'élimination de portion d'enrobage, la portion d'enrobage (20) est éliminée en faisant entrer en collision l'une contre l'autre la pluralité des portions de cœur (10) sur lesquelles la portion d'enrobage (20) est formée.

14. Procédé de fabrication d'un matériau de traitement d'absorption d'eau (1) selon la revendication 12 ou 13,
dans lequel dans l'étape d'élimination de portion d'enrobage, la portion d'enrobage (20) est éliminée en faisant se frotter l'une contre l'autre la pluralité des portions de cœur (10) sur lesquelles la portion d'enrobage (20) est formée.
